# EUROPEAN PATENT SPECIFICATION

(11) **EP 1 933 807 B1**
(45) Date of publication and mention of the grant of the patent: **04.11.2015**
(21) Application number: 06778537.8
(22) Date of filing: 11.08.2006
(51) Int. Cl.: A61Q 5/00, A61K 36/31, A61K 8/37, A61Q 5/02, A61K 8/97, A61K 36/14, A61Q 5/12

(54) **COSMETIC PRODUCT FOR THE TREATMENT OF SCALP**
KOSMETISCHES PRODUKT ZUR BEHANDLUNG DER KOPFHAUT
PRODUIT COSMETIQUE POUR LE TRAITEMENT DU CUIR CHEVELU

(30) Priority: 12.08.2005 FI 20055433
(43) Date of publication of application: 25.06.2008
(73) Proprietor: Cutrin Oy, 02780 Espoo (FI)
(72) Inventor: MÄKINEN, Päivi, FI-02760 Espoo (FI); ISOHANNI, Tiina, FI-02400 Kirkkonummi (FI)
(74) Representative: Matilainen, Mirja Helena
(86) International application number: PCT/FI2006/050354
(87) International publication number: WO 2007/020328

(56) References cited:
- FR-A1- 2 558 727
- FR-A1- 2 559 390
- FR-A1- 2 694 694
- RU-C1- 2 089 210
- RU-C1- 2 120 278
- RU-C1- 2 187 996
- US-A- 5 057 502
- Dr. Anthony Speroni: "Gemmotherapy", , 30 August 2012 (2012-08-30), XP002699040, Retrieved from the Internet: URL:http://www.drsperoni.com/gemmotherapy. htm [retrieved on 2013-06-18]
- POURMORTAZAVI S. ET AL.: 'Extraction of volatile compounds from Juniperus communis L. leaves with supercritical fluid carbon dioxide: comparison with hydrodistillation' FLAVOUR AND FRAGRANCE JOURNAL vol. 19, 2004, pages 417 - 420, XP008076892
- DAMJANOVIC B. ET AL.: 'A Comparison Between the Oil, Hexane Extract and Supercritical Carbon Dioxide Extract of Juniperus Communis L' JOURNAL OF ESSENTIAL OIL RESEARCH vol. 15, 2003, pages 90 - 92, XP008076886
- KING J.W.: 'Supercritical Fluid Processing of Cosmetic Raw Materials' COSMETICS AND TOILETRIES vol. 106, 1991, pages 61 - 64, XP008076873
- GRöNQVIST N.: 'Aromtech: Sense the Artic Spring' SOFW JOURNAL, ENGLISH EDITION vol. 132, no. 4, 2006, page 110, XP008076885

## Description

The object of this invention is a cosmetic product which can be used for the treatment of scalp, that is for the treatment or prevention of a greasy scalp and/or of dandruff. Specifically, the object of the invention is a hair shampoo or a hair conditioner formulation for said use, which can be of a type to be rinsed off or to be left on the scalp, such as a hair tonic, serum or lotion, or a mask.

Juniper belongs to the family of cypress plants (Cupressaceae). Juniper is spread over the whole Finland. The juniper growing in the southern and central Finland is usually of the bush-like title strain (Juniperus communis) and north of these areas mainly low or ground hugging Lappish juniper (Juniperus nana). In the area of the title strain also the tree-like (var. suecica) and the pillar-like (var. hibernica) juniper grows and in the maritime islands the ground hugging strain.

Many species of juniper are known, and extracts and distillates obtained therefrom have been used for cosmetic purposes (Lawless, J., The Encyclopedia of Essential Oils, p. 113).

Juniper, that is juniper berries, shoots, wood material, bark and ethereal oil, has been used as drugs in natural medicine.

In natural medicine juniper is considered to be a plant with i.a. inflammation removing and antimicrobial properties (Lawless, J, ibid; and Raipala-Courmier, Virpi, Luontoäidin kotiapteekki, kasvilääkintä ja luontaishoidot ("Nature Mother's Home Pharmacy, Plant Medicine and Natural Treatments"), p. 119). Juniper, especially dried berries and needled branches, has been proposed for use as a urinary and colic drug, as an antiseptic, stimulating and blushing substance, as well as externally as a relief of rheumatic pains, in the treatment of wounds, and for the prevention of hair loss (Stuart, M., Kauneutta ja Terveyttä Luonnonyrteistä, p. 209).

In cosmetics juniper has been used as a berry extract (Juniperus communis fruit extract) or as oil (Juniperus communis fruit oil; International Cosmetic Ingredient Dictionary and Handbook Monographs, 10 ed. 2004, p. 925). Juniper berry extract is antiseptic and aromatic, regulates secretion of sebum and stimulates hair and scalp. It also has a stimulating effect on the capillary circulation of scalp (brochure: Gattefosse Vegetal Genevrier GR 482 hydro).

Within this disclosure, a sprout or shoot means the annual new growth of a tree branch. Juniper sprouts or shoots contain ethereal oil (0.15 - 0.18%), resin, tans, wax, and flavonoids (rutin and xylosides) (Raipala-Cormier, V., ibid). In natural medicine, sprouts have been used as water baths, wherein the active agents have mainly been water-soluble. Ethereal oil contains monoterpenes (pinene, myrsene, sabinene, limonene, cymene, terpinene, tujene and camphene). According to the invention even the non-volatile, fat-soluble substances (waxes and waxy substances) can for the first time be utilized.

According to the invention it has now been discovered that juniper sprout extract has advantageous skin conditioning properties which enable its use especially on the scalp but also in the treatment and prevention of problems elsewhere on the skin, such as excessive sebum excretion, acne, and dandruff.

The object of the invention is especially a cosmetic product for the treatment of hair and scalp, which product in addition to one or several, cosmetically acceptable carrier and/or auxiliary agent(s) contains juniper sprout extract prepared by supercritical carbon dioxide extraction. A further object of the invention is the use of such juniper sprout extract for the treatment of scalp, especially for curing and prevention of its greasiness and scaling.

Juniper sprout extract differs from other raw materials obtainable from various parts of juniper due to its composition and also due to its preparation process. Due to the supercritical carbon dioxide extraction, the pure fat-soluble fractions of juniper sprout, such as waxes and ethereal oils, can for the first time be utilized in cosmetics, without the by-products and remainders of solvent which follow from the usual isolation processes, such as solvent extraction.

According to one preferred embodiment the product according to the invention contains 0.001 - 10% by weight of juniper sprout extract, especially 0.01 - 3% by weight, based on the total weight of the product.

The juniper sprout extract for use according to the invention is prepared by supercritical carbon dioxide extraction, which extraction method is well known to a person skilled in the art for the extraction and isolation of various substances. Juniper sprout extract is prepared by SFE technique for example by Aromtech Oy, Tornio, Finland.

Due to the low treatment temperature of the supercritical carbon dioxide extraction used for the preparation of juniper sprout extract also such components, e.g. waxes, are obtained in the extract, which e.g. by distillation cannot be utilized. As no solvents are used in supercritical extraction, the extract thus prepared is especially suitable for cosmetic purposes.

In a preferred embodiment, supercritical extraction is preferably carried out with dried juniper sprouts and pressurized carbon dioxide of almost room temperature. In supercritical state, carbon dioxide can dissolve e.g. fats extremely well, and thus it can be used for the extraction of various fat-soluble substances and oils, for example from sprouts and berries. The raw material and the end product, the extract, are in a fully airless state. In the process the sensitive biological active agents are maintained intact, as the low temperature and the carefully controlled process prevent oxidation and other detrimental reactions. If needed, some carbon dioxide can be left in the product to further protect from oxidation. The end products do not contain remainders of solvents, as environmentally detrimental chemicals are not used in any process step. This is naturally an advantage as it is a question of a cosmetic product to be used for hygiene.

The composition of a juniper sprout extract prepared by supercritical extraction differs e.g. from the composition of a juniper berry distillate. For this reason certain basic studies have been carried out for the new raw material, especially concerning the potential irritating effect of the raw material, its potential sensitizing effect and its potential cytotoxicity. Based on the tests, juniper sprout raw material prepared by supercritical extraction was not found to possess such properties that would prevent its use as a cosmetic raw material.

The product according to the invention can be any such product which can be used for the treatment of scalp, such as various shampoos and conditioners, including both those which are left on the hair and those which are rinsed off, such as hair tonics, emulsions and masks.

Dandruff, greasiness, erythema, and itching are common and unpleasant cosmetic problems of the scalp. Cosmetic anti-dandruff products usually contain an antimicrobial agent which prevents reproduction of yeast fungi of the genus Malassezia sp, the cause of dandruff. Malassezia furfur yeast fungus is commonly known also under its synonym name Pityrosporum Ovale (Mycology Online, School of Molecular and Biomedical Science, The University of Adelaide, Australia).

Known product forms used in the cosmetic treatment of dandruff are rinse-off shampoos and conditioners. Tonics, serums and lotions which are left on the scalp complement the effect of a dandruff shampoo. Products in emulsion form for peeling the dandruff of the scalp are sometimes needed. In such products, a mildly peeling compound, e.g. salicylate and a dandruff softening oil, e.g. mineral oil, such as paraffin oil or a vegetable oil, e.g. soya oil, are combined with an antimicrobial agent.

According to the invention it has been found that juniper sprout extract brings additional effectiveness also to cosmetic anti-dandruff products. Likewise, it has been found that juniper sprout extract brings additional effectiveness also to cosmetic products used for a greasy and blemished complexion.

The cosmetic product according to the invention contains one or several carrier and/or auxiliary agents accepted in the field of cosmetics. Such a cosmetic product can be for example a shampoo, a hair conditioner, such as a tonic, lotion, serum or an emulsion product, e.g. a peeling emulsion product.

Shampoos intended for washing and treatment of hair and scalp contain a solvent, usually washing agents in addition to water, such as sodium lauryl ether sulphate, thickeners, such as sodium chloride, auxiliary tensides which have an effect on the foaming and skin compatibility of the shampoo, e.g. betaine derivatives, a preservative, e.g. parabens or 5-bromo-5-nitro-1,3-dioxane, emulgators, caring, softening and moisturizing agents, such as e.g. emollients, such as PEG-7 glyceryl cocoate, other vegetable extracts than juniper shoot extract, or other raw materials from nature, such as e.g. tar or peat extract, agents that formulate product appearance, such as colours, pearly substances, acidity regulating agents, such as citric or lactic acid or bases, such as NaOH, by which the acidity of the product has been adjusted to pH 4-7, perfumes and ethereal oils.

Conditioners usually contain a solvent, which usually is water, conditioning agents, such as cationic tensides, such as cetrimonium chloride, thickeners, such as cetyl stearyl alcohol, a preservative, such as parabens, emulgators, conditioning, softening and moisturizing agents, such as e.g. emollients, vegetable extracts, agents formulating product appearance, such as colours, pearly substances, acidity regulating agents, such as citric or lactic acid or bases, such as NaOH, by which the acidity of the product has been adjusted to pH 3-5, perfumes and ethereal oils.

Tonics, serums and lotions which are left on the scalp generally contain a solvent, which most commonly is water, an auxiliary solvent which usually is ethanol, a preservative, e.g. parabens, emulgators, conditioning, softening and moisturizing substances, such as e.g. emollients, vegetable extracts and cationic tensides, thickeners, agents formulating product appearance, such as colours, acidity regulating agents, such as citric or lactic acid or bases, such as NaOH, by which the acidity of the product has been adjusted to pH 4-7, perfumes and ethereal oils.

Emulsion-like conditioner products usually contain a solvent, which most commonly is water, conditioning agents, such as cationic tensides, thickeners, such as cetyl stearyl alcohol, a preservative, e.g. parabens, thickeners, emulgators, conditioning, softening and moisturizing agents, such as e.g. emollients, vegetable extracts or other raw materials of nature, such as e.g. tar or peat extract, agents formulating product appearance, such as colours, acidity regulating agents, such as citric or lactic acid or bases, such as NaOH, by which the acidity of the product has been adjusted to pH 4-7, perfumes and ethereal oils. Emulsion-like conditioner products may further contain various components acceptable in the field of cosmetics, such as for example surface active agents. As to its composition, an emulsion may be an oil-in-water (o/w); water-in-oil (w/o) or water-oil-water (w/o/w).

Peeling agents, such as salicylates, enzymatic peeling agents, such as e.g. papaine or subtilisin, dandruff softening agents, such as vegetable oils or mineral oils, can be added to an emulsion product to obtain a peeling conditioning product in emulsion form. The amount of such peeling and softening agents is preferably 0.1-20% by weight.

According to one preferred embodiment of the invention a cosmetic product, such as a shampoo or a conditioner, may in addition to juniper sprout extract contain another active agent, especially an antimicrobial agent against dandruff. Such an antimicrobial agent is for example piroctone olamine, climbazole, and zinc pyrithione. The product according to the invention contains preferably 0.001 - 1% by weight of one or several such other active agent, based on the total weight of the product. A cosmetic product for dandruff may in addition contain various components accepted in the field of cosmetics, such as for example scalp refreshing and stimulating agents, such as camphor, menthol, peppermint oil, and tea tree oil.

The following examples illustrate the invention.
I. The shampoo composition according to the invention may contain for example

| | |
|---|---|
| a) water | ad 100% |
| b) detergent | 5-20 % by weight |
| c) auxiliary tensides | 0.5-15 % by weight |
| d) preservative | 0.01-1 % by weight |
| e) emollients | 0.1-10 % by weight |
| f) juniper sprout extract prepared by supercritical extraction | 0.001-10 % by weight |
| g) other components and auxiliary agents | 0.001-30 % by weight |

The shampoo composition according to the invention contains juniper sprout extract together with an appropriate washing composition. A treatment with this composition results in a clear reduction of problems of the scalp. If desired, an anti-dandruff antimicrobial agent can be added to said composition, a suitable amount being 0.01-1% by weight, based on the total composition.
IA. A clarifying example of the dandruff shampoo composition having the above composition is as follows:

| | |
|---|---|
| a) water | ad 100% |
| b) sodium alkyl ether sulphate 2 EO/3 EO | 10-15 % by weight |
| (or partly 8 EO) | |
| c) amphoteric surface active agents (undecylene amido propyl betaine or cocamidopropyl betaine) | 0.2-1.5 % by weight |
| non-ionic surface active agents (PEG 200 hydrogenated glyceryl palmate, PEG 7 glyceryl cocoate) | 0.1-5 % by weight |
| d) Piroctone Olamine | 0.001-1 % by weight |
| e) preservative, e.g. 5-bromo-5-nitro-1,3-dioxane | 0.01-0.2 % by weight |
| f) emollients (tridecyl salicylate, C12-C13 alkyl lactate) | 0.2-5 % by weight |
| g) juniper sprout extract prepared by supercritical extraction | 0.001-10 % by weight |
| h) emulgators (PEG 40 Hydrogenated castor oil, PEG-36 castor oil) | 0.1-5 % by weight |
| i) perfume | 0.1-1 % by weight |
| k) colour | 0.01-1 % by weight |
| l) acidity regulating agent, e.g. citric acid, for adjusting acidity of the product to the range pH 4-7 | 0.01-1 % by weight |
| m) thickening agents (NaCl) | 0.01-4 % by weight |

II. A conditioner composition according to the invention may contain for example

| | |
|---|---|
| a) water | ad 100% |
| b) cationic tenside | 01-10% by weight |
| c) emulgator and thickening agents | 0.1-10 % by weight |
| d) preservative | 0.01-1 % by weight |
| e) emollients | 0.1-10 % by weight |
| f) juniper sprout extract prepared by supercritical extraction | 0.001-10 % by weight |
| g) other components and auxiliary agents | 0.001-30 % by weight |

The conditioner composition according to the invention contains juniper sprout extract together with a suitable cationic conditioner composition. As a result of the treatment with the composition, the problems of scalp are clearly reduced. If desired, an anti-dandruff antimicrobial agent can be added to the composition, a suitable amount being 0.01-1 % by weight, based on the total composition.
IIA. A clarifying example of the dandruff treatment composition having the above composition is as follows:

| | |
|---|---|
| a) water | ad 100% |
| b) alkyl trimethyl ammonium chloride and cationic metosulphate, e.g. Distearoyl ethyl hydroxyethyl monium methosulfate | 0.1-10 % by weight |
| c) ceteareth -20, cetearyl alcohol, cocamide MEA | 0.1-10 % by weight |
| d) Piroctone Olamine | 0.001 - 1 % by weight |
| e) preservative, e.g.. parabens | 0.01-0.5 % by weight |
| f) emollients (tridecyl salicylate, C12-C13 alkyl lactate) | 0.2 -5 % by weight |
| g) juniper sprout extract prepared by supercritical extraction | 0.001-10 % by weight |
| h) other vegetable extracts, e.g. olive oil and wax and their derivatives | 0.1-5 % by weight |
| i) perfume | 0.1-1 % by weight |
| k) colour | 0.01-1 % by weight |
| l) acidity regulating agent, e.g. citric acid, for regulating acidity of the product to the range pH 3-5 | 0.01-1 % by weight |
| m) thickening agents (NaCl) | 0.01-4 % by weight |

III. A hair tonic/lotion/serum composition according to the invention may contain

| | |
|---|---|
| a) water | ad 100% |
| b) ethanol | 0.1-80 % by weight |
| c) thickening agents | 0.1-10 % by weight |
| d) emollients | 0.1-10 % by weight |
| e) juniper sprout extract prepared by supercritical extraction | 0.001-10 % by weight |
| f) other components and auxiliary agents | 0.001-30 % by weight |

A hair tonic/lotion/serum composition according to the invention contains juniper sprout extract together with a suitable scalp caring composition. As a result of the treatment with the composition, the problems of the scalp are clearly reduced. If desired, an anti-dandruff antimicrobial agent can be added, a suitable amount being 0.01 - 1 % by weight based on the total composition.
IIIA. A clarifying example of an anti-dandruff hair tonic/lotion/serum composition having the above composition is as follows:
An anti-dandruff hair tonic/lotion/serum composition

| | |
|---|---|
| a) water | ad 100% |
| b) ethanol | 10-65 % by weight |
| c) refreshing and stimulating agents, such as menthol | 0.1-1% by weight |
| d) Piroctone Olamine | 0.001-1% by weight |
| e) propylene glycol | 0.01-10% by weight |
| f) emollients (Tridecyl Salicylate, C12-C13 Alkyl lactate) | 0.2-5 % by weight |
| g) juniper sprout extract prepared by supercritical extraction | 0.001-10 % by weight |
| h) emulgators (PEG 40 Hydrogenated castor oil, PPG-26-buteth-26) | 0.1-5 % by weight |
| i) perfume | 0.1-1 % by weight |
| k) colour | 0.01-1 % by weight |
| l) acidity regulating agent, e.g. citric acid, for adjusting acidity of the product to the range pH 4-7 | 0.01-1 % by weight |

IV. An emulsion-like conditioner composition according to the invention may contain:

| | |
|---|---|
| a) water | ad 100% |
| b) emulgator and thickening agents | 0.1-10 % by weight |
| c) preservative | 0.01-1 % by weight |
| d) emollients | 0.1-10 % by weight |
| e) juniper sprout extract prepared by supercritical extraction | 0.001-10 % by weight |
| f) other components and auxiliary agents | 0.001-30 % by weight |

The composition of the emulsion-like product according to the invention contains juniper sprout extract together with an emulsion-like composition. As a result of treatment with the conditioner composition, the problems of the scalp are clearly reduced. If desired, also a peeling agent and an anti-dandruff antimicrobial agent can be added to the emulsion product, wherein a suitable amount of the first mentioned agent is preferably 0.001-10% by weight and the amount of the antimicrobial agent is 0.01-1% by weight.
IVA. A peeling, anti-dandruff emulsion product of the above mentioned type is as follows:

| | |
|---|---|
| a) water | ad 100% |
| b) ceteareth-3, glyceryl stearate | 0.1-10% by weight |
| c) Piroctone Olamine | 0.001-1 % by weight |
| d) preservative, e.g. parabens | 0.01-0.5% by weight |
| e) emollients (tridecyl-salicylate, C12-C13 alkyl lactate) | 0.2-5 % by weight |
| g) juniper sprout extract prepared by supercritical extraction | 0.001-10 % by weight |
| h) other vegetable extracts, e.g. olive oil and wax and their derivatives, mineral and vegetable oils, especially paraffin oil and rapeseed oil (Canola oil), solvent (propylene glycol) | 0.1-35 % by weight |
| i) perfume | 0.1-1 % by weight |
| k) colour | 0.01-1% by weight |
| l) acidity regulating agent, e.g. citric acid, for adjusting acidity of the product to the range pH 4-7, and antioxidants, e.g. tocopherol and ascorbyl palmitate | 0.001-1 % by weight |

### Experimental

Both separate raw material combinations (juniper sprout extract combined with emollients, with piroctone olamine, and with surface active agents) and end products (shampoos, lotions, and conditioners) have been tested according to the fungicidal effect test applied by the Department of Publich Health (Kansanterveystieteen laitos, KTTL) of the University of Helsinki (fungicidal effect test EN 1650, dandruff fungus Malasezzia furfur, test strain ATCC 96809). In the studies of KTTL, fungistatic effect according to standard EN 1650 was found with the shampoo that contained juniper sprout extract prepared by supercritical extraction method.

The shampoo according to the invention has been tested both in studio, hairdresser's and consumer tests for about 3 months. Further, these products have been tested in use by voluntary test persons suffering from scalp disorders of various degrees. Testing has been done under supervised conditions of use under the evaluation of an expert in the field of hair (Cutrin Studio). In connection with this test, the change in the condition of the scalp has been followed by means of a scalp camera. In the tests, the dandruff shampoo composition according to example IA was used, together with a hair tonic composition according to the invention, corresponding to the composition mentioned in example IIIA, and which have been tested against severe scalp scaling of a psoriatic. Already after one week treatment, a clear reduction of scaling on the scalp was detected, and after two week scaling started to be under control. After three weeks a remarkable progress compared to the initial situation was found, in other words scaling was hardly at all seen on the scalp.

According to the results of use tests, 75% of hairdressers included in the study estimated that the test product reduced itching, erythema and was from excellently to well suitable for a scaling scalp. 25% of hairdressers found the result satisfactory and none considered the result of treatment as weak. In the opinion of consumers, the test product reduced itching and was well suitable for a scaling scalp (100%). On the other hand, erythema was reduced well (60%).

## Claims

1. A cosmetic product for the treatment of scalp, **characterized in that** in addition to one or several cosmetically acceptable carrier and/or auxiliary agent(s) it contains juniper sprout extract prepared by supercritical carbon dioxide extraction.

2. The product according to claim 1, **characterized in that** it contains 0.001 - 10% by weight of juniper sprout extract, based on the total weight of the product.

3. The product according to claim 2, **characterized in that** it contains 0.01-3% by weight of juniper sprout extract, based on the total weight of the product.

4. The product according to any one of the above claims, **characterized in that** the juniper sprout extract is an extract prepared from Juniperus communis.

5. The product according to any one of the above claims, **characterized in that** it is intended for the treatment of scaling, greasiness, erythema, dandruff and/or itching of scalp.

6. The product according to any one of the above claims, **characterized in that** the product further contains one or several antimicrobial agent(s).

7. The product according to claim 6, **characterized in that** the antimicrobial agent is selected from the group consisting of piroctone olamine, climbazole, and zinc pyrithione.

8. The product according to claim 6 or 7, **characterized in that** said additive is present in the product in the amount of 0.001 - 1 % by weight, based on the total weight of the product.

9. The product according to any one of the above claims, **characterized in that** it is a hair shampoo or a hair conditioner.

10. The product according to claim 5 or 6, **characterized in that** it contains an emollient, especially tridecyl salicylate or C12-13 alkyl lactate.

11. The product according to claim 10, **characterized in that** it contains vegetable oil, especially rapeseed oil.

12. Non-therapeutic use of a juniper sprout extract prepared by supercritical carbon dioxide extraction for the treatment of scalp, especially for the treatment or prevention of a greasy scalp.

## Patentansprüche

1. Kosmetisches Produkt für die Behandlung der Kopfhaut, **dadurch gekennzeichnet, dass** es zusätzlich zu einem oder mehreren kosmetisch verträglichen Trägern und/oder Hilfsstoff(en) Wacholdersprossenextrakt enthält, der durch überkritische Kohlendioxidextraktion hergestellt wurde.

2. Produkt nach Anspruch 1, **dadurch gekennzeichnet, dass** es 0,001-10 Gew.-% Wacholdersprossenextrakt enthält, basierend auf dem Gesamtgewicht des Produkts.

3. Produkt nach Anspruch 2, **dadurch gekennzeichnet, dass** es 0,01-3 Gew.-% Wacholdersprossenextrakt enthält, basierend auf dem Gesamtgewicht des Produkts.

4. Produkt nach einem der vorangehenden Ansprüche, **dadurch gekennzeichnet, dass** der Wacholdersprossenextrakt ein aus Juniperus communis hergestellter Extrakt ist.

5. Produkt nach einem der vorangehenden Ansprüche, **dadurch gekennzeichnet, dass** es für die Behandlung von Abschuppung von Kopfhaut, fettiger Kopfhaut, geröteter Kopfhaut, Schuppen und/oder Juckreiz der Kopfhaut bestimmt ist.

6. Produkt nach einem der vorangehenden Ansprüche, **dadurch gekennzeichnet, dass** das Produkt des Weiteren einen oder mehrere antimikrobielle(n) Wirkstoff(e) enthält.

7. Produkt nach Anspruch 6, **dadurch gekennzeichnet, dass** der antimikrobielle Wirkstoff ausgewählt ist aus der Gruppe bestehend aus Pirokton-Olamin, Climbazol, und Zink-Pyrithion.

8. Produkt nach Anspruch 6 oder 7, **dadurch gekennzeichnet, dass** der Zusatzstoff in dem Produkt in einer Menge von 0,001-1 Gew.-% vorliegt, basierend auf dem Gesamtgewicht des Produkts.

9. Produkt nach einem der vorangehenden Ansprüche, **dadurch gekennzeichnet, dass** es ein Haarshampoo oder eine Haarspülung ist.

10. Produkt nach Anspruch 5 oder 6, **dadurch gekennzeichnet, dass** es einen Weichmacher, insbesondere Tridecylsalicylat oder C12-13-Alkyl-Laktat, enthält.

11. Produkt nach Anspruch 10, **dadurch gekennzeichnet, dass** es pflanzliches Öl, insbesondere Rapsöl, enthält.

12. Nicht-therapeutische Verwendung eines durch überkritische Kohlendioxidextraktion hergestellten Wacholdersprossenextrakts für die Behandlung der Kopfhaut, insbesondere für die Behandlung oder Vorbeugung von fettiger Kopfhaut.

## Revendications

1. Produit cosmétique destiné au traitement du cuir chevelu, **caractérisé en ce que**, en plus d'un ou plusieurs excipients et/ou agents auxiliaires acceptables du point de vue cosmétique, il contient de l'extrait de pousse de genévrier préparé par extraction au dioxyde de carbone supercritique.

2. Produit selon la revendication 1, **caractérisé en ce qu'**il contient de 0,001 à 10 % en poids d'extrait de pousse de genévrier, sur la base du poids total du produit.

3. Produit selon la revendication 2, **caractérisé en ce qu'**il contient de 0,01 à 3 % en poids d'extrait de pousse de genévrier, sur la base du poids total du produit.

4. Produit selon l'une quelconque des revendications précédentes, **caractérisé en ce que** l'extrait de pousse de genévrier est un extrait préparé à partir de Genévrier commun.

5. Produit selon l'une quelconque des revendications précédentes, **caractérisé en ce qu'**il est destiné au traitement des squames, de la graisse, de l'érythème, des pellicules et/ou des démangeaisons du cuir chevelu,

6. Produit selon l'une quelconque des revendications précédentes, **caractérisé en ce que** le produit contient en outre un ou plusieurs agents antimicrobiens.

7. Produit selon la revendication 6, **caractérisé en ce que** l'agent antimicrobien est sélectionné à partir du groupe constitué par la piroctone olamine, le climbazole, et le pyrithione de zinc.

8. Produit selon la revendication 6 ou 7, **caractérisé en ce que** ledit additif est présent dans le produit en une quantité de 0,001 à 1 % en poids, sur la base du poids total du produit.

9. Produit selon l'une quelconque des revendications précédentes, **caractérisé en ce qu'**il est un shampooing ou un conditionneur pour cheveux.

10. Produit selon la revendication 5 ou 6, **caractérisé en ce qu'**il contient un émollient, en particulier, du salicylate de tridécyle ou du lactate d'alkyle en C₁₂ à C₁₃.

11. Produit selon la revendication 10, **caractérisé en ce qu'**il contient de l'huile végétale, en particulier, de l'huile de colza.

12. Utilisation non thérapeutique d'un extrait de pousse de genévrier préparé par extraction au dioxyde de carbone supercritique afin d'assurer le traitement du cuir chevelu, en particulier, le traitement ou la prévention d'un cuir chevelu graisseux.
